# EUROPÄISCHE PATENTANMELDUNG

(11) **EP 1 438 977 A2**
(43) Veröffentlichungstag der Anmeldung: **21.07.2004**
(21) Anmeldenummer: 03028450.9
(22) Anmeldetag: 12.12.2003
(51) Int. Cl.: A61M 5/00, A61M 5/31

(54) **Sterilhülle für eine Injektionsspritze**

(30) Priorität: 17.01.2003 DE 10301884
(71) Anmelder: Aventis Behring Gesellschaft mit beschränkter Haftung, 35002 Marburg (DE); Maslanka, Herbert, 78532 Tuttlingen (DE)
(72) Erfinder: Moersdorf, Gerhard, 67728 Münchweiler/Alsenz (DE); Maslanka, Herbert, 78532 Tuttlingen (DE)

(57) **Zusammenfassung**

Die Erfindung betrifft eine Sterilhülle (1) für eine Injektionsspritze (4).

Die Erfindung schlägt vor, dass die Sterilhülle aus einer verschließbaren Umhüllung aus Kunststoff besteht, sie ferner einen Ausgabestutzen (2) aufweist, wobei das Innere der Sterilhülle der Aufnahme der Injektionsspritze, die mit dem Ausgabestutzen verbindbar ist, dient und der Ausgabestutzen mit einem Ventil (15) versehen ist, mit Durchflussrichtung ausschließlich von der Injektionsspritze nach außen.

Eine solche Sterilhülle für eine Injektionsspritze gewährleistet eine optimale Sterilität des von der Injektionsspritze aufgenommenen Produkts. Wegen des Ventils können keine Keime von außen in die Spritze aufsteigen.

## Beschreibung

Die Erfindung betrifft eine Sterilhülle für eine Injektionsspritze. Mit einer solchen sterilen Hülle kann eine Injektionsspritze für mehrere Applikationen keimfrei gehalten werden.
Das mittels einer Injektionsspritze dem Patienten zu verabreichende Arzneimittel ist gelegentlich so reichlich bemessen, dass nicht der ganze Inhalt der Injektionsspritze erforderlich ist. Die Aufbewahrung des in der Injektionsspritze verbliebenen restlichen Arzneimittels für eine spätere Verwendung bei demselben oder einem anderen Patienten ist jedoch nur dann möglich, wenn die Injektionsspritze bis dahin steril aufbewahrt wird.

Eine Sterilhülle für eine Injektionsspritze ist aus der DE 299 20 664 U1 bekannt. Die Sterilhülle besteht aus einem durchsichtigen und verschließbaren Plastikbeutel, der der Aufnahme der Injektionsspritze dient. Zum Applizieren des Medikaments ist der Plastikbeutel mit einer Einmalkanüle ausgerüstet. Das der Einmalkanüle gegenüberliegende Ende ist mittels eines Adhäsionsverschlusses verschließbar. Der Plastikbeutel ist geeignet, zwei Injektionsspritzen zur Applikation eines Gewebeklebers aufzunehmen. Eine Injektionsspritze enthält eine Fibrinogenlösung und die andere eine Thrombinlösung und es ist für jede dieser beiden Lösungen eine eigene Kanüle oder eine gemeinsame Doppelkanüle vorgesehen.

Bei einer solchen Sterilhülle mit Injektionsspritze und Einmalkanüle besteht die Gefahr einer aufsteigenden mikrobiellen Kontamination, indem Baktieren vom Kanülenende her in die Injektionsspritze aufsteigen. Bei bestimmungsgemäßen Gebrauch, der die sofortige Entfernung der Injektionsspritze nach Applikation vorsieht, stellt dies kein Problem dar.

Aufgabe der Erfindung ist es, eine Sterilhülle für eine Injektionsspritze zu schaffen, die eine optimale Sterilität des von der Injektionsspritze aufgenommenen Produkts gewährleistet.

Zur Lösung dieser Aufgabe schlägt die Erfindung eine Sterilhülle für eine Injektionsspritze mit folgenden Merkmalen vor:
- die Sterilhülle besteht aus einer verschließbaren Umhüllung aus Kunststoff,
- die Sterilhülle weist einen Ausgabestutzen auf,
- das Innere der Sterilhülle dient der Aufnahme der Injektionsspritze, die mit dem Ausgabestutzen verbindbar ist,
- der Ausgabestutzen ist mit einem Ventil versehen, mit Durchflussrichtung von der Injektionsspritze nach außen.

Wesentlich ist bei der Sterilhülle, dass deren Ausgabestutzen zusätzlich mit dem Ventil versehen ist. Dies gewährleistet dem Anwender eine möglichst große Anwendungssicherheit. Selbst dann, wenn er nicht unmittelbar nach der Applikation die Injektionsspritze aus dem Ausgabestutzen der Sterilhülle entfernt, stellt dies kein Problem dar, weil wegen des Ventils keine Bakterien von außen in die Spritze aufsteigen können.

Mit dem äußeren Bereich des Ausgabestutzens ist ein medizinisches Gerät verbindbar, insbesondere eine Kanüle, ein Adapter, ein Mehrwegehahn oder eine Infusionsflasche.

Die Umhüllung ist insbesondere, zumindest bereichsweise, durchsichtig. Selbstverständlich kann sie, um die von der Sterilhülle umschlossene Injektionsspritze gut einsehen und handhaben zu können, auch vollständig durchsichtig sein.

Die Durchflussrichtung durch das Ventil von der Injektionsspritze nach außen kann auf unterschiedlich Art und Weise bewerkstelligt werden: Vorzugsweise ist das Ventil als Rückschlagventil oder Entenschnabelventil ausgebildet. Grundsätzlich ist es denkbar, ein in beiden Richtungen offenes Ventil vorzusehen, wie es bei Infusionsflaschen verwendet wird. Ein solches, in beiden Richtungen offenes Ventil hat einen Druckkörper in der Mitte, der nur bei Überschreiten eines bestimmten Druckes öffnet. Dieser Ventiltyp kann deshalb Anwendung finden, weil die Drucküberschreitung nur bei Einwirkung der Injektionsspritze erfolgt, nicht aber durch das mit dem Ausgabestutzen verbindbare medizinische Gerät, beispielsweise die Kanüle.

Gemäß einer bevorzugten Ausgestaltung des Ventils ist vorgesehen, dass der Ausgabestutzen mindestens eine radiale Auslassöffnung aufweist, die mittels eines den Ausgabestutzen umschließenden elastischen Ringelements, insbesondere eines schlauchförmigen Ringelements, verschließbar ist.

Zweckmäßig dient der Ausgabestutzen sowohl der Aufnahme der Injektionsspritze als auch des medizinischen Gerätes, insbesondere der Kanüle. Bei Verwendung handelsüblicher Injektionsspritzen weist der Ausgabestutzen eine konusförmige Ausnehmung auf, die der Aufnahme des Spritzenkonus der Injektionsspritze dient. Bei Verwendung einer üblichen Einmalkanüle weist der Ausgabestutzen einen als Konus ausgebildeten Abschnitt zur Aufnahme einer konusförmigen Ausnehmung der Einmalkanüle auf. Andererseits kann eine Drehverschluss zum Verbinden des Ausgabestutzens mit dem medizinischen Gerät vorgesehen sein, beispielsweise in Art eines Luer-Lock-Verschlusses oder eines Gewindes. Es weist somit der Ausgabestutzen einen Gewindeabschnitt zur Aufnahme eines Gewindeabschnittes des medizinischen Gerätes auf.

Gemäß einer besonderen Gestaltung ist vorgesehen, dass der Ausgabestutzen als Hohlkörper ausgebildet ist. Die Abmessungen des Hohlkörpers sind vorzugsweise so bemessen, dass er im wesentlichen der Aufnahme des Zylinderabschnitts der Injektionsspritze dient. Der Hohlkörper weist damit im wesentlichen Zylinderform auf. Der sich bei der Injektionsspritze quer zum Spritzenkolben erstreckende Griff ist außerhalb des Hohlkörpers angeordnet, so dass er günstig ergriffen werden kann.

Um das Produkt applizieren zu können, ist die Sterilhülle in dem dem Ausgabestutzen abgewandten Bereich folienartig, somit flexibel ausgebildet, so dass die Injektionsspritze in üblicher Art und Weise gehandhabt werden kann.

Gemäß einer besonderen Ausführungsform der Erfindung ist vorgesehen, dass die Sterilhülle durch den Ausgabestutzen und einen Druckbeutel gebildet ist. Der steife Ausgabestutzen ist insbesondere als Spritzgussteil aus Kunststoff ausgebildet. Auch der Druckbeutel sollte aus Kunststoff bestehen und ist beispielsweise als Spritzgussteil gestaltet sein. Allerdings wird es als vorteilhaft angesehen, wenn der Druckbeutel durch ein Ansatzstück, insbesondere ein steifes Ansatzstück, zum Verbinden mit dem Ausgabestutzen und eine folienartige Kunststoffkappe gebildet ist.

In Abhängigkeit von den produzierten Stückzahlen der erfindungsgemäßen Sterilhülle kann der Druckbeutel, statt als Kunststoffspritzgussteil, auch in einem Tauchverfahren oder durch Blasextrusion hergestellt werden.

Um eine sichere, lösbare Verbindung von Ansatzstück und Ausgabestutzen zu gewährleisten, ist das Ansatzstück vorzugsweise mit einem Rastvorsprung, insbesondere einem umlaufenden Rastvorsprung versehen, der in Hintergriff mit einem entsprechenden Rastvorsprung einer ringförmigen Platte des Ausgabestutzens bringbar ist.

Aufgrund der Gestaltung der Injektionsspritze, mit sich senkrecht zur Verschieberichtung des Spritzenkolbens erstreckenden Griffstegen, wird es als vorteilhaft angesehen, wenn der Ausgabestutzen im Bereich seiner ringförmigen Platte, die dem Ventil abgewandt ist, eine ovale Ringöffnung aufweist, und der Druckbeutel, insbesondere dessen Ansatzstück, im wesentlichen ein Oval umschließt.

Vorteilhaft ist zusätzlich ein Dichtelement, das beispielsweise als Dichtlippe ausgebildet ist, vorgesehen, um im Verbindungsbereich von Ausgabestutzen und Druckbeutel abzudichten.

Weitere Merkmale der Erfindung sind in den Schutzansprüchen, der Beschreibung der Figuren und den Figuren selbst dargestellt.

In der Zeichnung der Figuren ist die Erfindung anhand eines Ausführungsbeispiels, bei Verwendung einer Einmalkanüle als medizinischem Gerät, veranschaulicht, ohne hierauf beschränkt zu sein. Es zeigt:

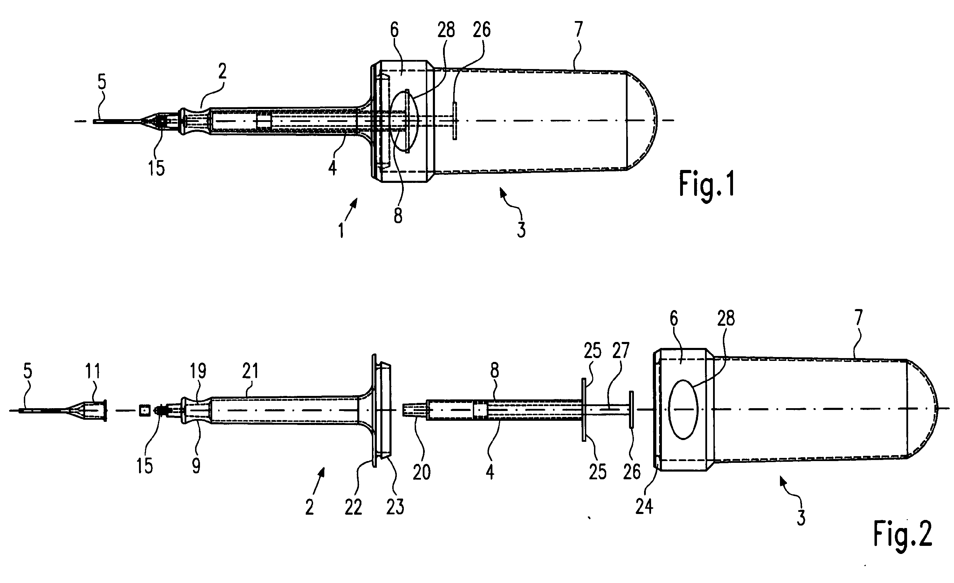

- Figur 1: die erfindungsgemäße Sterilhülle mit Injektionsspritze und Einmalkanüle, im montierten Zustand, von der Seite gesehen,
- Figur 2: der Aufbau der Sterilhülle mit Injektionsspritze und Einmalkanüle in einer Ansicht gemäß Figur 1, allerdings einer Explosionsdarstellung,
- Figur 3: eine vergrößerte Darstellung des Ausgabestutzens, von der Seite gesehen,
- Figur 4: eine Einzelheit Z des in Figur 3 veranschaulichten Ausgabestutzens,
- Figur 5: den Bestandteil der Sterilhülle bildenden Druckbeutel, in einer Seitenansicht,
- Figur 6: den in Figur 5 gezeigten Druckbeutel im Bereich des Anschlusses an den Ausgabestutzen,
- Figur 7: den Druckbeutel in einer Ansicht VII gemäß Figur 5 und
- Figur 8: den Ausgabestutzen in einer Ansicht VIII gemäß Figur 3.

Die Sterilhülle 1 ist durch einen Ausgabestutzen 2 und einen mit dieser rastierend verbindbaren Druckbeutel 3 gebildet. Bezogen auf die Funktion der Sterilhülle 1 ist das Innere des Ausgabestutzens 2 eine Injektionsspritze 4 und außen auf den Ausgabestutzen 2 eine Einmalkanüle 5 steckbar. Der Ausgabestutzen 2 stellt ein steifes Bauteil dar, dass aus Kunststoff besteht und im Spritzguss hergestellt ist. Auch der Druckbeutel 3 ist als Kunststoffspritzgussteil ausgebildet. Er ist durch ein steifes Ansatzstück 6 und eine mit diesem verbundene folienartige Kunststoffkappe 7 gebildet. Das Ansatzstück 6 ist rastierend mit dem Ausgabestutzen 2 verbindbar.

Aufgrund der flexiblen Gestaltung der Kunststoffkappe 7 kann von außen die Injektionsspritze 4 betätigt werden, ohne diese unmittelbar ergreifen zu müssen.

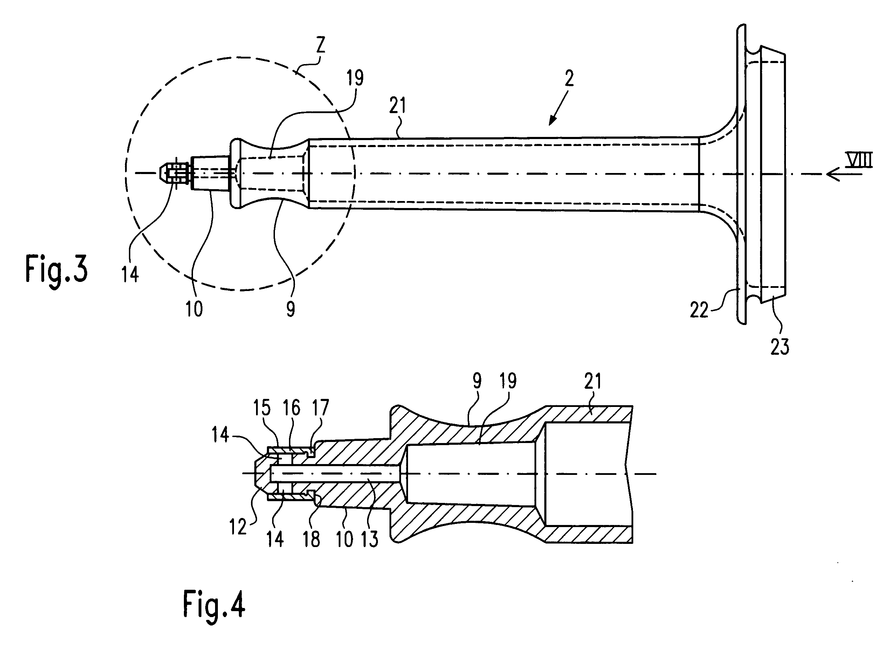

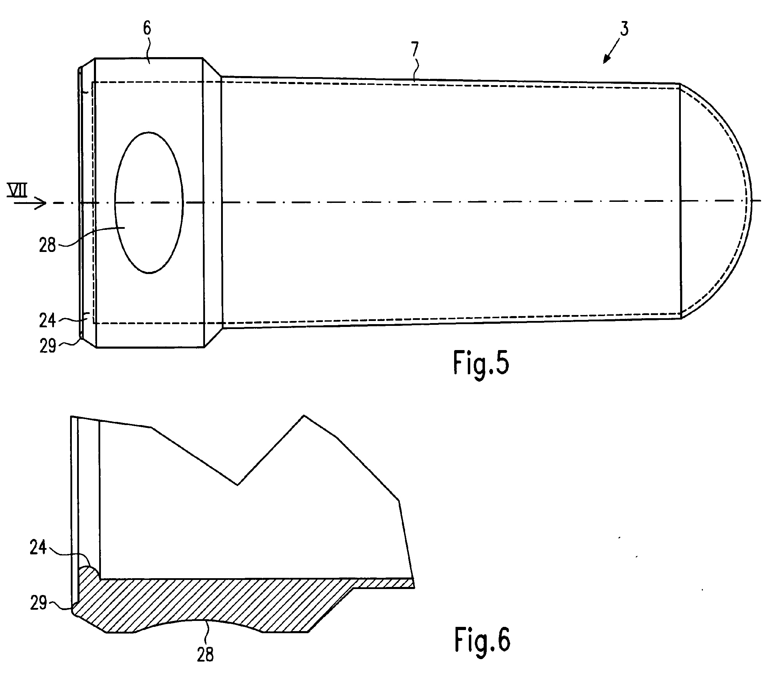

Der Ausgabestutzen 2 ist als Hohlkörper ausgebildet, der im wesentlichen der Aufnahme des Zylinderabschnitts 8 der Injektionsspritze 4 dient. In seinem vorderen Bereich weist der Ausgabestutzen 2, wie es insbesondere der Darstellung der Figuren 3 und 4 zu entnehmen ist, eine umlaufende Griffmulde 9 auf, an die sich, nach vorne, ein konisch zulaufender Abschnitt 10 zur Aufnahme einer konusförmigen Ausnehmung 11 der Einmalkanüle 5 anschließt. Der Abschnitt 10 und ein noch weiter vorn angeordneter Abschnitt 12 des Ausgabestutzens 2 sind mit einer endständig verschlossenen Axialbohrung 13 versehen, die im Bereich des Abschnitts 12 in eine diesen radial durchsetzende Bohrung 14 mündet. Die beiden Bohrungsöffnungen der Bohrung 14 sind mittels eines Rückschlagventils 15 verschlossen. Dieses weist einen flexiblen Kunststoffring 16, beispielsweise aus Silikon, auf, der flächig den Mantel des Abschnitts 12 umschließt und die Bohrung 14 mit Vorspannung abdeckt. Der Kunststoffring 16 weist benachbart dem Abschnitt 10 einen umlaufenden, nach innen gerichteten Nasenabschnitt 17 auf, der in eine Hinterschneidung 18 des Abschnitts 12 eingreift und damit als axiale Sicherung des Kunststoffrings 16 fungiert.

Wird durch die Bohrungen 13 und 14 ein Medikament, beispielsweise Fibrinogen oder Thrombin appliziert, hebt sich der Kunststoffring 16 geringfügig vom Abschnitt 12 ab und es öffnet damit das Rückschlagventil 15. In entgegengesetzte Richtung kann wegen der Funktion des Rückschlagventils 15 das Medikament nicht gelangen.

Im Bereich der Griffmulde 9 ist der Ausgabestutzen 2 innen mit einer konusförmigen Ausnehmung 19 versehen, die der Aufnahme des Spritzenkonus 20 der Injektionsspritze 4 dient. Der Innendurchmesser des hohlzylindrischen Abschnittes 21 des Ausgabestutzens 2 ist so bemessen, dass er ausreichend größer ist als der Außendurchmesser des Zylinderabschnitts 8 der Injektionsspritze 4, so dass diese mit Spiel in den Abschnitt 21 des Ausgabestutzens 2 eingeführt und mit ihrem Spritzenkonus 20 in die Ausnehmung 19 des Ausgabestutzens 2 eingesteckt werden kann.

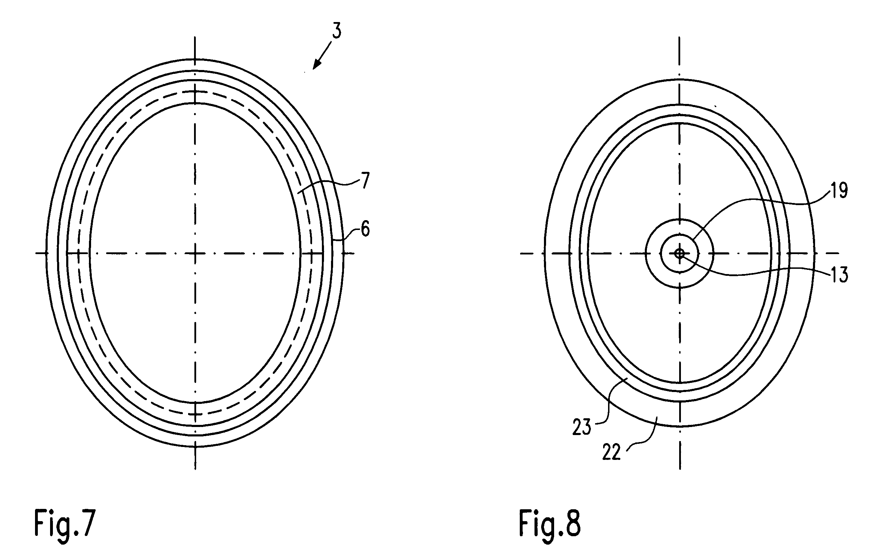

Das dem Abschnitt 12 abgewandte Ende des Ausgabestutzens 2 ist gegenüber dem Abschnitt 21 des Ausgabestutzens 2 deutlich erweitert ausgebildet und in Art einer ringförmigen Platte 22 gestaltet. Wie der Darstellung der Figur 8 zu entnehmen ist, ist die Platte 22 oval gestaltet und mit einem entsprechend oval positionierten Rastvorsprung 23 versehen. Dieser ist in Wirkverbindung mit einem Rastvorsprung 24 des Ansatzstückes 6 des Druckbeutels 3 bringbar. Insofern ist auch das Ansatzstück 6 oval ausgebildet, wie es der Darstellung der Figur 7 zu entnehmen ist. Eine Baueinheit mit dem Ansatzstück 6 bildet die flexible Kunststoffkappe 7. - Die ovale Gestaltung der vorbeschriebenen Bauteile berücksichtigt die radiale Ausdehnung der Injektionsspritze 4 im Bereich der mit dem Zylinderabschnitt verbundenen diametralen Grifflaschen 25. Mit der Bezugsziffer 26 ist die Grifflasche bezeichnet, die mit der Kolbenstange 27 der Injektionsspritze 4 verbunden ist. Im Bereich der beiden Längsseiten ist das Ansatzstück 6, um die Sterilhülle 1 insgesamt günstiger greifen zu können, mit Griffmulden 28 versehen.

Im Bereich des Nasenabschnittes 17 und der Hinterschneidung 18 des Ausgabestutzens 2 ergibt sich eine Dichtung zwischen dem Kunststoffring 16 des Rückschlagventils 15 und den Abschnitten 10 und 12 des Ausgabestutzens 2. Eine weitere Dichtung ist zwischen dem Ansatzstück 6 und der ringförmigen Platte 22 des Ausgabestutzens 2 durch eine umlaufende Dichtlippe 29 des Ansatzstückes 6 gebildet.

Die beschriebene Sterilhülle 1 besteht somit aus zwei dicht miteinander zu verbindenden Spritzgussteilen. Am Auslassende ist das Rückschlagventil 15 angebracht, das nur bei vorher (bei der Konstruktion) eingestelltem Druck öffnet und nach Applikation einen Rückfluss in die Injektionsspritze 4 verhindert. Die Einmalkanüle 5 kann über einen normalen Konus oder über einen Luer-Lock-Anschluss angedockt werden. Der Anwender ist frei in der Wahl der Kanüle. Die Applikation des Medikamentes ist nur bei einem entsprechend ausgeübten Druck auf die Kolbenstange 27 der Injektionsspritze 4 möglich. Die Stärke des erforderlichen Druckes zum Öffnen des Rückschlagventils 15 kann durch Änderung beispielsweise des Durchmessers der Auslassbohrung 14, Stärke / Elastizität des abdichtenden Kunststoffrings 16, Art des Ventils usw. voreingestellt werden. Das Ventil verhindert ein Aufsteigen von Flüssigkeit und damit Keimen.

### Bezugszeichenliste

- Sterilhülle: 1
- Ausgabestutzen: 2
- Druckbeutel: 3
- Injektionsspritze: 4
- Einmalkanüle: 5
- Ansatzstück: 6
- Kunststoffkappe: 7
- Zylinderabschnitt: 8
- Griffmulde: 9
- Abschnitt: 10
- Ausnehmung: 11
- Abschnitt: 12
- Bohrung: 13
- Bohrung: 14
- Rückschlagventil: 15
- Kunststoffring: 16
- Nasenabschnitt: 17
- Hinterschneidung: 18
- Ausnehmung: 19
- Spritzenkonus: 20
- Abschnitt: 21
- Platte: 22
- Rastvorsprung: 23
- Rastvorsprung: 24
- Grifflasche: 25
- Grifflasche: 26
- Kolbenstange: 27
- Griffmulde: 28
- Dichtlippe: 29

## Patentansprüche

1. Sterilhülle (1) für eine Injektionsspritze (4) mit folgenden Merkmalen:
- die Sterilhülle (1) besteht aus einer verschließbaren Umhüllung aus Kunststoff,
- die Sterilhülle (1) weist einen Ausgabestutzen (2) auf,
- das Innere der Sterilhülle (1) dient der Aufnahme der Injektionsspritze (4), die mit dem Ausgabestutzen (2) verbindbar ist,
- der Ausgabestutzen (2) ist mit einem Ventil (15) versehen, mit Durchflussrichtung ausschließlich von der Injektionsspritze (4) nach außen.

2. Sterilhülle nach Anspruch 1, wobei der äußere Bereich des Ausgabestutzens (2) der Aufnahme eines medizinischen Gerätes, insbesondere einer Kanüle (5), eines Adapters, eines Mehrwegehahnes oder einer Infusionsflasche dient.

3. Sterilhülle nach Anspruch 1 oder 2, wobei die Umhüllung zumindest bereichsweise durchsichtig ist.

4. Sterilhülle nach einem der Ansprüche 1 bis 3, wobei das Ventil (15) als Rückschlagventil oder Entenschnabelventil ausgebildet ist.

5. Sterilhülle nach Anspruch 4, wobei der Ausgabestutzen (2) mindestens eine radiale Auslassöffnung (14) aufweist, die mittels eines den Ausgabestutzen (2) umschließenden elastischen Ringelements (16), insbesondere eines schlauchförmigen Ringelements verschließbar ist.

6. Sterilhülle nach einem der Ansprüche 1 bis 5, wobei der Ausgabestutzen (2) eine konusförmige Ausnehmung (19) zur Aufnahme eines Spritzenkonus (20) der Injektionsspritze (4) aufweist.

7. Sterilhülle nach einem der Ansprüche 1 bis 6, wobei der Ausgabestutzen (2) als Hohlkörper ausgebildet ist, der im wesentlichen der Aufnahme des Zylinderabschnitts (8) der Injektionsspritze (4) dient.

8. Sterilhülle nach einem der Ansprüche 1 bis 7, wobei der Ausgabestutzen (2) im Bereich seines dem Ventil (15) abgewandten Endes als ringförmige Platte (22), insbesondere als Platte mit ovaler Ringöffnung ausgebildet ist.

9. Sterilhülle nach einem der Ansprüche 1 bis 8, wobei der Ausgabestutzen (2) als steifes Kunststoffspritzgussteil ausgebildet ist.

10. Sterilhülle nach einem der Ansprüche 1 bis 9, die durch den Ausgabestutzen (2) und einen Druckbeutel (3) gebildet ist.

11. Sterilhülle nach Anspruch 10, wobei der Druckbeutel (3) als Kunststoffspritzgussteil ausgebildet ist.

12. Sterilhülle nach Anspruch 10 oder 11, wobei der Druckbeutel (3) durch ein Ansatzstück (6) zum Verbinden mit dem Ausgabestutzen (2), insbesondere ein steifes Ansatzstück, und eine folienartige Kunststoffkappe (7) gebildet ist.

13. Sterilhülle nach Anspruch 12, wobei das Ansatzstück (6) mit einem Rastvorsprung (24) versehen ist, der im Hintergriff mit einem Rastvorsprung (23) der ringförmigen Platte (22) des Ausgabestutzens (2) bringbar ist.

14. Sterilhülle nach einem der Ansprüche 10 bis 13, wobei ein Dichtelement (29) zum Abdichten von Ausgabestutzen (2) und Druckbeutel (3) vorgesehen ist.

15. Sterilhülle nach einem der Ansprüche 2 bis 14, wobei der Ausgabestutzen (2) einen als Konus ausgebildeten Abschnitt (10) zur Aufnahme einer konusförmigen Ausnehmung (11) des medizinischen Gerätes (5) aufweist.

16. Sterilhülle nach einem der Ansprüche 2 bis 14, wobei der Ausgabestutzen (2) und das medizinische Gerät mittels eines Drehverschlusses verbindbar sind.
